(19) Europäisches Patentamt
European Patent Office
Office européen des brevets

(11) Publication number: **0 168 658**
**A1**

# (12) EUROPEAN PATENT APPLICATION

(21) Application number: **85107529.1**

(22) Date of filing: **18.06.85**

(51) Int. Cl.[4]: **C 07 D 207/34**, A 61 K 31/40

(30) Priority: **19.06.84 IT 2147084**

(43) Date of publication of application: **22.01.86**
**Bulletin 86/4**

(84) Designated Contracting States: **AT BE CH DE FR GB IT LI LU NL SE**

(71) Applicant: **POLI INDUSTRIA CHIMICA S.p.A., Piazza Agrippa, 1, I-20141 Milano (IT)**

(72) Inventor: **Massaroli, Gian Giacomo, Via Lauro, Rovegnate Como (IT)**

(74) Representative: **Bianchetti, Giuseppe, Studio Consulenza Brevettuale Via Senato 24, I-20121 Milan (IT)**

(54) **Pyrrolecarboxylic acid derivative having analgesic activity preparation thereof and compositions therefrom.**

(57) 5-(4-Fluorobenzoyl)-1,2,4-trimethyl-3-pyrrolcarboxylic acid
of formula I

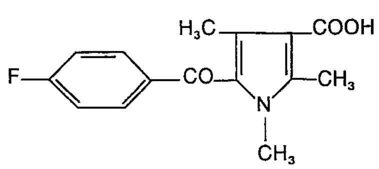

(I)

and its non toxic salts with inorganic cations or deriving from organic bases or aminoacids, are endowed with central analgesic activity are substantially devoid of side effects.

EP 0 168 658 A1

0168658



# PYRROLECARBOXYLIC ACID DERIVATIVE HAVING ANALGESIC ACTIVITY PREPARATION THEREOF AND COMPOSITIONS THEREFROM

The present invention refers to 5-(4-fluoro-benzoyl)-1,2,4-trimethyl-3-pyrrolcarboxylic acid having formula I

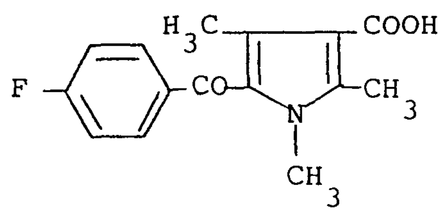

(I)

and its non-toxic salts with inorganic cations or deriving from organic bases or aminoacids.

Another object of the invention is provided by a process for preparing the compound I.

A further object of the invention is provided by pharmaceutical composition having analgesic activity comprising, as the active principle, the compound of formula I or its pharmaceutically acceptable salts.

Pyrrolacetic acid derivatives endowed with analgesic activity are known from DE-A-2,102,746. In particular, 5-(p-chlorobenzoyl)-1,4-dimethylpyrrole-2-acetic acid, also known as zomepirac, proved to be an effective analgesic agent and it is presently used in human therapy.

A major problem connected with the clinical use of zomepirac, is due to its capacity of inhibiting the enzymes cyclooxygenase and prostaglandine-synthetase. As a result of this enzymatic inhibition, zomepirac, as well as other analgesic-antiinflammatory agents, exhibits ulcerogenic properties and may induce anaphylactoid reactions as

a consequence of the formation of leukotrienes.

The compound I which, differently from zomepirac, is a pyrrolcarboxylic acid derivative instead of a pyrrolacetic acid derivative, is surprisingly devoid of cyclooxygenase and prostaglandine-synthetase inhibiting activity and it is therefore devoid of ulcerogenic and anaphylactoid properties. Moreover, the compound of the invention does not induce addiction effects, differently from other centrally acting analgesics and particularly from the opioid analgesics (morphine, codeine, etc.).

According to the present invention the compound of formula I may be conveniently prepared according to the following reaction scheme:

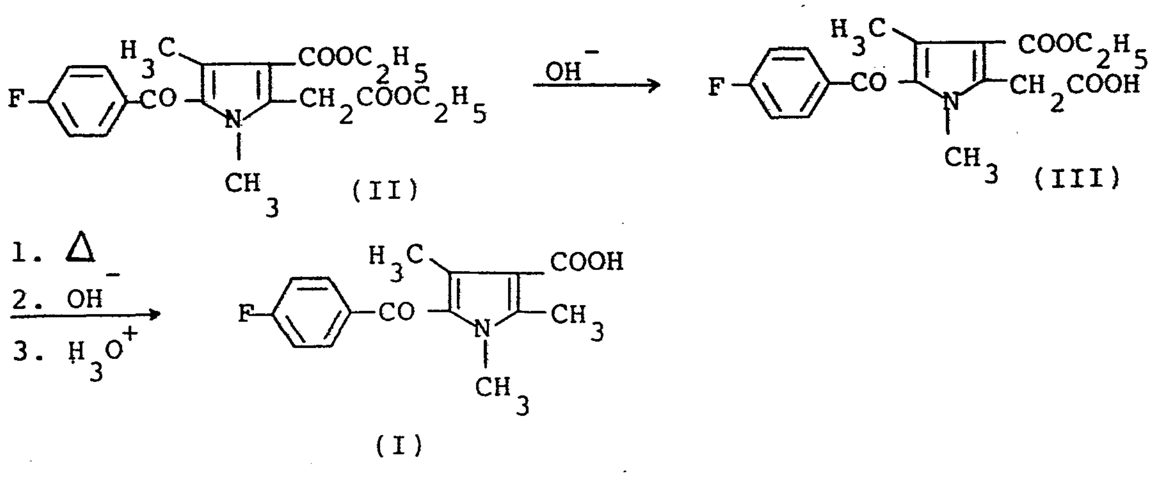


(II) (III) (I)

The mono-saponification of the diester II with equimolar amounts of sodium hydroxide in aqueous isopropanol occurs selectively and with good yields at the carboxymethyl group linked in position 2 of the pyrrole nucleus. The reaction course is unequivocally confirmed by the NMR spectrum of compound I, which clearly shows the presence of a methyl group in position 2 of the pyrrole ring. The following example illustrates the invention

without limiting the scope thereof.

## EXAMPLE

a) 1,4-Dimethyl-5-(4-fluorobenzoyl)-3-ethoxycarbonylpyrrole-2-acetic acid (III)

56.25 Grams (0.15 moles) of ethyl-1,4-dimethyl-5-(4-fluorobenzoyl)-3-ethoxycarbonylpyrrole-2-acetate were suspended in 300 ml of an isopropanol-water (80:20) mixture. The reaction mixture was refluxed and 75 ml (0.15 moles) of a 2N sodium hydroxide aqueous solution was added dropwise thereto, during 30 minutes. The reflux was kept for 1 hour, the isopropanol was distilled off, the mixture was diluted with water, acidified with HCl and extracted several times with ethyl acetate. The ethyl acetate solution was concentrated and cooled, obtaining the crystallization of 43 g (82.6% yield) of a product having m.p. 159-161°C.

b) 5-(4-Fluorobenzyl)-1,2,4-trimethyl-3-pyrrolecarboxylic acid I

30 Grams (0.0864 moles) of the product obtained in a) were heated to 200°C for 3 hours in nitrogen stream. After cooling, the residue was taken up with 100 ml of ethanol and 100 ml of 15% sodium hydroxide aqueous solution and the mixture was refluxed for 3 hours. The ethanol was evaporated, water was added to the mixture, the aqueous solution was heated at 60°C and strongly acidified with hydrochloric acid. After cooling, the precipitated crude acid was filtered, washed with water, dried and crystallized from ethyl acetate, obtaining 19 g (80% yield) of a product melting at 197-198° C.

The analgesic activity of the compound of formula I

by the oral route, has been determined in the mouse using the following tests:

- Phenylquinone test (Hendershot and Forsaith - J. Pharmacol. Exptl. Ther. 123, 237 (1959));
- Hot-plate test. Male albino mice, treated with increasing doses of the compound under exam, are placed on a plate thermostatized at 55°C. An effective dose 50 ($ED_{50}$) is defined as the dose inducing a 50% increase of the reaction time to the pain stimulus.

The acute toxicity *per os* is expressed as $LD_{50}$ at the $14^{th}$ observation day.

As a comparison codeine hydrochloride was used.

The results are reported in the following Table.

**TABLE I**

| | Phenylquinone $ED_{50}$ mg/kg | Hot place $EX_{50}$ mg/kg | $LD_{50}$/mouse os mg/kg |
|---|---|---|---|
| Compound I | 2.1 | 3.5 | >1600 |
| Codeine HCl | 5.6 | 25 | 300 |

The compound of formula I, differently from codeine hydrochloride, does not induce the phenomenon known as "jumping by nalorphine" in the mouse, which rules out the possibility of provoking physical addiction. Moreover, the compound I unequivocally differentiates from known compounds having similar structure, such as zomepirac, in that it is endowed with central analgesic activity without cyclooxygenase and prostaglandine-synthetase inhibitory activity, with the consequent and above mentioned advantages.

An essential aspect of the invention is provided by pharmaceutical formulations containing predetermined amount of compound I, useful for the treatment of acute and chronic pain of different nature, particularly deriving from neoplasias, surgical operations, head-ache, tooth-ache etc.

The compound of the present invention can be administered by the oral, rectal or parenteral route, for instance in form of capsules, coated-tablets, tablets, syrups, drops, suppositories, microclisms, phials.

The following formulations are cited by way of an example:

a) **tablets** containing from 25 to 500 mg of the compound I, with excipients and disgregating agents commonly used in the pharmaceutical technique;

b) **suppositories** containing from 50 to 1000 mg of compound I;

c) **vials** for intramuscular or endovenous use containing from 25 to 500 mg of compound I, in form of a salt thereof, at a pH of about the physiological values.

CLAIMS for the Contracting States:
BE, CH, DE, FR, GB, IT, LI, LU, NL, SE

1. 5-(4-Fluorobenzoyl)-1,2,4-trimethyl-3-pyrrolcarboxylic acid of formula I

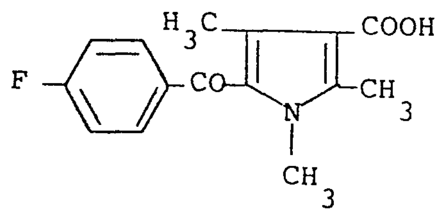

(I)

and its non toxic salts with inorganic cations or deriving from organic bases or aminoacids.

2. Process for the preparation of the compound I characterized in that ethyl 1,4-dimethyl-5-(4-fluorobenzoyl)-3-ethoxycarbonylpyrrole-2-acetate is hydrolyzed to 1,4-dimethyl-5-(4-fluorobenzoyl)-3-ethoxycarbonylpyrrole-2-acetic acid which is then decarboxylated by means of hot alkali.

3. Pharmaceutical composition having analgesic activity containing as the active principle the compound of formula I or one of the salt thereof, in admixture with pharmaceutically acceptable carriers and excipients.

4. Compositions according to claim 3 for the oral administration in form of capsules, tablets, syrups, drops containing from 25 to 500 mg by unit dose of the compound I or the equivalent of salts thereof.

5. Compositions according to claim 3 suited for the administration by rectal route in form of suppositories and microclisms containing from 50 to 1000 mg of compound

I or the equivalent of salts thereof.

6. Compositions according to claim 3 suited for the parenteral administration in form of vials or bottles containing from 25 to 500 mg of the compound I or the equivalent of salts thereof.

CLAIMS for AT

1. Process for the preparation of 5-(4-Fluorobenzoyl)-1,2,4-trimethyl-3-pyrrolcarboxylic acid of formula I

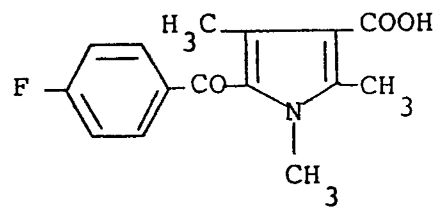

(I)

and its non toxic salts with inorganic cations or deriving from organic bases or aminoacids, characterized in that ethyl 1,4-dimethyl-5-(4-fluorobenzoyl)-3-ethoxycarbonyl-pyrrole-2-acetate is hydrolyzed to 1,4-dimethyl-5-(4-fluorobenzoyl)-3-ethoxycarbonylpyrrole-2-acetic acid which is then decarboxylated by means of hot alkali.

European Patent Office

# EUROPEAN SEARCH REPORT

0168658 Application number

EP 85 10 7529

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | EP-A-0 001 534 (A. ROLLAND S.A.)<br>* Whole document *<br>--- | 1-6 | C 07 D 207/34<br>A 61 K 31/40 |
| A | US-A-4 282 242 (HOLLAND)<br>* Columns 1-3 *<br>----- | 1-6 | |
| | | | TECHNICAL FIELDS SEARCHED (Int. Cl.4) |
| | | | C 07 D 207/00 |

The present search report has been drawn up for all claims

| Place of search | Date of completion of the search | Examiner |
|---|---|---|
| THE HAGUE | 11-10-1985 | MAISONNEUVE J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82